# EUROPEAN PATENT SPECIFICATION

(11) **EP 4 541 364 B1**
(45) Date of publication and mention of the grant of the patent: **07.10.2026**
(21) Application number: 24206797.3
(22) Date of filing: 15.10.2024
(51) Int. Cl.: A61K 36/736, A23L 2/38, A23L 33/105, A61P 1/10, C12P 1/04, C12R 1/865, C12P 1/02

(54) **PEACH BLOSSOM FERMENTATION LIQUID FOR IMPROVING CONSTIPATION, AND PREPARATION METHOD AND USE THEREOF**
PFIRSICHBLÜTENFERMENTATIONSFLÜSSIGKEIT ZUR VERBESSERUNG VON VERSTOPFUNG SOWIE HERSTELLUNGSVERFAHREN UND VERWENDUNG DAVON
LIQUIDE DE FERMENTATION DE LA PÊCHE À LA FLEURS POUR AMÉLIORER LA CONSTIPATION, SON PROCÉDÉ DE PRÉPARATION ET SON UTILISATION

(30) Priority: 20.10.2023 CN 202311373515
(43) Date of publication of application: 23.04.2025
(73) Proprietor: Fung-wong Pharmaceutical Group (Zhuhai) Co., Ltd, Zhuhai, Guangdong 519099 (CN); South China Agricultural University, Guangzhou, Guangdong 510642 (CN)
(72) Inventor: LI, Pan, Guangzhou, 510642 (CN); LIANG, Shan, Guangzhou, 510642 (CN); DU, Bing, Guangzhou, 510642 (CN); LIANG, Ziping, Guangzhou, 510642 (CN); HE, Zhipeng, Guangzhou, 510642 (CN)
(74) Representative: Bayramoglu et al.

(56) References cited:
- CN-A- 105 746 706
- CN-A- 112 369 593
- CN-A- 115 227 808
- FU YU ET AL: "Exploring the Active Constitutions of Peach Blossom for Amelioration of Loperamide-Induced Mice Constipation by Ultra-Performance Liquid Chromatography-Mass Spectrometry Combined with Network Pharmacology", vol. 18, no. 3, 1 March 2023 (2023-03-01), US, XP093252022, ISSN: 1934-578X, Retrieved from the Internet <URL:https://journals.sagepub.com/doi/full-xml/10.1177/1934578X231161184> DOI: 10.1177/1934578X231161184

## Description

### TECHNICAL FIELD

The present invention relates to the field of traditional Chinese medicine processing, and in particular to a peach blossom fermentation liquid for improving constipation, and a preparation method and use thereof.

### BACKGROUND

Constipation is a common gastrointestinal disease, the main symptoms of which are difficulty in defecation, hard stools, decreased defecation frequency, and decreased gastrointestinal motility, etc. The constipation is broadly divided into functional constipation, organic constipation and intractable constipation. The functional constipation in turn includes temporary conventional constipation caused by reduced food and water intake or consumption of foods low in dietary fibers, and frequent and habitual slow transit constipation. The pathogenesis of the slow transit constipation is complex, mainly caused by factors such as slow colon transit, an abnormal enteric nervous system, smooth muscle abnormalities, abnormal gastrointestinal peptide hormones, great mental stress, and abuse of laxatives.

An opioid drug is one of the factors that cause the slow transit constipation. The opioid drug is often used in the analgesic treatment of a cancer patient. Excessive use of it can cause adverse responses such as constipation, nausea and vomiting, and muscular rigidity. The constipation is a typical adverse response of the opioid drug. The pathogenesis of the slow transit constipation induced by the opioid drug is that: the opioid drug acts directly on the central nervous system and the gastrointestinal smooth muscle, to inhibit the central nervous system so that the defecation reflex is insensitive, the gastrointestinal motility is decreased, the gastric emptying is delayed, and the reflex between the anal sphincter and rectal distension is damaged, thereby aggravating constipation. Compared with the temporary conventional constipation, the slow transit constipation caused by the opioid drug has more complex pathogenesis and more severe symptoms, and is more difficult to cure, so it has higher requirements for treatment drugs.

Currently, the drug treatment for the slow transit constipation induced by the opioid drug usually uses laxatives, aperients, stool softeners, gastrointestinal motility drugs or peripheral receptor antagonists and so on. However, these medicines have significant side effects. Long-term use of them causes drug dependence, melanosis coli, intestinal mucosal damage and the like disadvantages, which on the contrary aggravate constipation. Moreover, there are some reports of using traditional Chinese medicine compositions to make decoctions, ointments, capsules or oral solutions to relieve slow transit constipation caused by opioid drugs. The relatively common medicinal materials in these traditional Chinese medicine compositions are fleeceflower root, Chinese angelica, rhubarb, Magnolia officinalis, immature orange fruit, Largehead Atractylodes Rhizome, Cannabis Fructus, Areca catechu, etc. These traditional Chinese medicine compositions can play a certain role in improvement. However, due to the different medicinal properties of each traditional Chinese medicine in the compositions, although some medicinal materials can relieve some symptoms, excessive use of some medicinal materials may cause other functional intestinal diseases such as diarrhea. Additionally, since the traditional Chinese medicines themselves taste bitter, combining them will undoubtedly further amplify the bad smell, making it difficult for patients to swallow it when administration and causing psychological repellence of the patients.

Therefore, it has become a technical problem to be urgently solved by those skilled in the art how to provide a product with good sensory organs and clear and significant efficacy in relieving slow transit constipation caused by the opioid drugs.

CN115227808 discloses a plant fermentation product comprising yam, Poria, peach blossom, angelica, honey, Alisma and Astragalus.

CN112369593 discloses a peach blossom flavored condiment, prepared by crushing fresh peach blossoms into a slurry, passing them through a 20/30/40-mesh sieve, adding pectinase and cellulase for enzymatic hydrolysis, adding yeast and glucose as a carbon source for anaerobic fermentation for 12-20 h, and adding peach gum to the fermentation mixture.

Fu et al., "Exploring the Active Constitutions of Peach Blossom for Amelioration of Loperamide-Induced Mice Constipation by Ultra-Performance Liquid Chromatography-Mass Spectrometry Combined with Network Pharmacology", Natural Product Communications, 18(3) reveals a peach blossom extract, obtained by pulverizing dry peach blossoms, reflux extracting 20 g of powder with 200 mL of distilled water at 100°C for 1 h twice, filtering and combining the two filtrates, evaporating and freeze-drying the final product.

### SUMMARY

In the present discloure, a technical problem to be solved is to overcome the defect in the prior art that there is a lack of products with good sensory organs and clear and significant efficacy in relieving slow transit constipation caused by opioid drugs, thereby providing a peach blossom fermentation liquid for improving constipation, and a preparation method and use thereof.

To achieve the aforementioned objective, the present invention provides the following technical solutions.

On the one hand, the present invention provides a method for preparing a peach blossom fermentation liquid, including the following steps:
(1) soaking dried peach blossoms in water and then decocting to obtain a peach blossom liquid with dregs;
(2) adding a flavourzyme and a cellulase to the peach blossom liquid with dregs for enzymolysis, and inactivating the enzymes, then obtaining an enzymolysis dreg-liquid mixture;
(3) adding a carbon source into the enzymolysis dreg-liquid mixture, and sterilizing to obtain a fermentation substrate;
(4) inoculating *Saccharomyces cerevisiae* into the fermentation substrate for a primary fermentation, and sterilizing after the fermentation is completed to obtain a primary fermentation liquid; and
(5) inoculating composite lactic acid bacteria into the primary fermentation liquid for a secondary fermentation, sterilizing after the fermentation is completed, filtering, and concentrating to obtain the peach blossom fermentation liquid, wherein the composite lactic acid bacteria comprise *Lactobacillus plantarum, Lactobacillus bulgaricus, Lactobacillus helveticus, Leuconostoc mesenteroides subsp. mesenteroides,* and *Streptococcus thermophilus.*

Further, in the step (4),
*Saccharomyces cerevisiae* powder is added into the fermentation substrate for the primary fermentation, and the added amount of the *Saccharomyces cerevisiae* powder is 1%-7% by mass of the fermentation substrate;
the conditions of the primary fermentation are: a temperature of 30 ± 2°C, a rotation speed of 120 r/min, and a time of 4 d; and
the conditions of the sterilizing are 85°C and 20 min.

Further, *Saccharomyces cerevisiae* powder is added into the fermentation substrate for the primary fermentation, and the added amount of the *Saccharomyces cerevisiae* powder is 3% by mass of the fermentation substrate.

Further, in the step (5),
a composite lactic acid bacteria liquid is added into the primary fermentation liquid for a secondary fermentation, the added amount of the composite lactic acid bacteria liquid is 2%-8% by volume of the fermentation substrate; in the composite lactic acid bacteria liquid, the viable bacteria count of the *Lactobacillus plantarum* is 1.5 × 10⁸-3 × 10⁸ cfu/mL; the viable bacteria count of the *Lactobacillus bulgaricus* is 2 × 10⁶ to 4 × 10⁶ cfu/mL; the viable bacteria count of the *Lactobacillus helveticus* is 1 × 10⁶-2×10⁶ cfu/mL; the viable bacteria count of the *Leuconostoc mesenteroides subsp. mesenteroides* is 1 × 10⁸ to 2 × 10⁸ cfu/mL; and the viable bacteria count of the *Streptococcus thermophilus* is 2 × 10⁶ to 4 × 10⁶ cfu/mL;
the conditions of the secondary fermentation are: a temperature of 33 ± 1°C, a speed of 120 r/min, and a time of 3 d;
the conditions of the sterilizing are 85°C and 20 min;
the concentrating is to concentrate the filtered filtrate to 1/3-1/2 of the original volume.

Further, a composite lactic acid bacteria liquid is added into the primary fermentation liquid for a secondary fermentation, the added amount of the composite lactic acid bacteria liquid is 4% by volume of the fermentation substrate; in the composite lactic acid bacteria liquid, the viable bacteria count of the *Lactobacillus plantarum* is 1.5 × 10⁸ cfu/mL; the viable bacteria count of the *Lactobacillus bulgaricus* is 3 × 10⁶ cfu/mL; the viable bacteria count of the *Lactobacillus helveticus* is 1 × 10⁶ cfu/mL; the viable bacteria count of the *Leuconostoc mesenteroides subsp. mesenteroides* is 1 × 10⁸ cfu/mL; and the viable bacteria count of the *Streptococcus thermophilus* is 3 × 10⁶ cfu/mL.

The composite lactic acid bacteria liquid is obtained by activating composite lactic acid bacteria powder in a MRS liquid medium; wherein in the composite lactic acid bacteria powder, a mass ratio of *Lactobacillus plantarum* powder, *Lactobacillus bulgaricus* powder, *Lactobacillus helveticus* powder, *Leuconostoc mesenteroides subsp. mesenteroides* powder, and *Streptococcus thermophilus* powder is 1:1.5:1:1:1.5.

Further, in the step (2), an enzymolysis temperature is 40-60°C; an enzymolysis time is 60-180 min; an added amount of the flavourzyme (flavor enzyme) is 0.5%-1.5% by mass of the peach blossom liquid with dregs; the added amount of the cellulase is 0.5%-2% by mass of the peach blossom liquid with dregs; and the conditions of inactivating the enzymes are 110°C and 20 min.

Preferably, an enzymolysis temperature is 55°C; an enzymolysis time is 120 min; the added amount of the flavourzyme is 1.5% by mass of the peach blossom liquid with dregs; and the added amount of the cellulase is 0.75% by mass of the peach blossom liquid with dregs.

Further, the step (1) includes: primary decoction: soaking the dried peach blossom in water, and then decocting and filtering to obtain a filtrate and a filter residue; and secondary decoction: taking the filter residue, adding with water and decocting again, filtering, and combining the filtrates and filter residues of the two times of decoction to obtain the peach blossom liquid with dregs,
in the step of primary decoction, the amount of the water added into the dried peach blossom is 20-40 times the mass of the dried peach blossom; a soaking time is 30-40 min; and the decoction process is sequentially as follows: decocting at 1,800-2,100 W for 20-40 min, decocting at 1,200-1,500 W for 20-40 min, and decocting at 600-800 W for 20-40 min;
in the step of secondary decoction, the amount of the water added into the filter residue is 10-20 times the mass of the dry peach blossom; and the decoction process is sequentially as follows: boiling at 1,800-2,100 W and then switching to decoct at 600-800 W for 40-60 min.

Preferably, in the step of primary decoction, the amount of water added into the dried peach blossoms is 20 times the mass of the dried peach blossom; a soaking time is 30 min; and the decoction process is sequentially as follows: decocting at 2,100 W for 20 min, decocting at 1,400 W for 20 min, and decocting at 800 W for 20 min;
in the step of secondary decoction, the amount of the water added into the filter residue is 10 times the mass of the dry peach blossom; and the decoction process is sequentially as follows: boiling at 2,100 W and then switching to decoct at 800 W for 40 min.

Further, in the step (3), the carbon source is white granulated sugar, and the added amount of the white granulated sugar is 3%-20% by mass of the enzymolysis dreg-liquid mixture; and the conditions of sterilizing are 80-100°C and 5-10 min.

Preferably, the added amount of the white granulated sugar is 5% by mass of the enzymolysis dreg-liquid mixture; and the conditions of sterilizing are 100°C and 5 min.

In another aspect, the present invention provides a peach blossom fermentation liquid obtained by the preparation method.

In another aspect, the present invention provides the peach blossom fermentation liquid obtained by the preparation method for use in for improving constipation.

Further, the constipation is temporary conventional constipation or slow transit constipation.

Further, the slow transit constipation is constipation caused by an opioid drug; and the opioid drug includes loperamide.

The technical solutions of the present invention have the following advantages:
Peach blossom is petals of a deciduous tree *Prunus persica* (L.) Batsch, *Amygdalus L.* of *Rosaceae,* and is bitter in taste, neutral in nature and non-toxic. The peach blossom is rich in active ingredients such as protein, naringenin, kaempferol, and the like. **In** the prior art, it can be used as raw materials for tea, vinegar, and wine, and can also be used as a raw material for cosmetics. The present invention uses peach blossom as a preparation raw material for the first time, and prepares a peach blossom fermentation liquid through a specific fermentation means, which has a clear effect on improving slow transit constipation caused by the opioid drug.

**In** the present invention, firstly active substances are dissolved from the peach blossom by soaking and decocting the peach blossom, and then further subjected to composite enzymolysis by the flavourzyme and the cellulase, so as to promote the conversion of the protein in the peach blossom liquid with dregs and the cellulose in plant cell walls into small molecule substances to participate in the subsequent fermentation process together, and at the same time, the flavourzyme can be utilized to hydrolyze flavor precursors, thereby removing the bitterness, releasing flavor substances, and improving the taste. The enzymolysis dreg-liquid mixture is then fermented in steps. The first step is to produce aromatic alcohols and some ester substances under the fermentation of *Saccharomyces cerevisiae,* so as to give the fermentation liquid a special mellow flavor, and at the same time, the fermentation liquid becomes clearer and more transparent under the action of *Saccharomyces cerevisiae.* The second step is to perform fermentation by the composite lactic acid bacteria, wherein *Streptococcus thermophilus* in the composite lactic acid bacteria utilizes the amino acid produced in the fermentation process of *Saccharomyces cerevisiae,* and at the same time, *Lactobacillus (Lactobacillus plantarum, Lactobacillus bulgaricus,* and *Lactobacillus helveticus)* further converts the residual protein into an amino acid for the growth of *Streptococcus thermophilus.* The rapid metabolism of *Streptococcus thermophilus* produces acidic substances such as lactic acid and formic acid, which can in turn promote the growth of *Lactobacillus.* At the same time, *Leuconostoc mesenteroides subsp. mesenteroides* can further promote the growth of *Lactobacillus.* Under the joint action of various strains, the color and lustre of the fermentation liquid are improved to a certain extent. At the same time, acidic substances such as acetic acid produced through decomposition by the composite lactic acid bacteria react with alcohol substances with a mellow aroma such as pentanol produced by the fermentation of *Saccharomyces cerevisiae* to produce ester substances such as ethyl valerate, which also reduces the content of substances with bitter almond aroma such as 2,4-dimethylbenzaldehyde and benzaldehyde in the peach blossom liquid before fermentation, giving the peach blossom fermentation liquid a unique fruity aroma and a sour-sweet and tasty taste. The peach blossom fermentation liquid obtained by adding *Saccharomyces cerevisiae* and the composite lactic acid bacteria at the same time for one-step fermentation has a higher content of alcohol substances, has basically no excess acid substances as produced, and has a strong bitter almond aroma and a poor taste.

In the present invention, the macromolecular substances in the peach blossom are converted into small molecular substances that can be directly absorbed by the intestinal tract of the body through means such as soaking, decocting, enzymolysis and fermentation, which is more conducive to the absorption by the body and greatly increases the content of nutrients and active ingredients in the peach blossom liquid. For example, the contents of soluble dietary fiber, total sugar, polysaccharide, total phenol, and total acid in the final peach blossom fermentation liquid are greatly improved, and the contents of phenolic and flavonoid ingredients such as chlorogenic acid, kaempferol, quercetin, rutin and hyperoside are also improved to varying degrees.

The peach blossom fermentation liquid prepared by the present invention has an increased content of soluble dietary fiber, can soften stool, increase the water content of mouse feces, and shorten the time to the first red stool excretion of mice. The phenolic substances in the fermentation liquid further stimulate gastrointestinal motility, accelerate gastric emptying and intestinal motility of mice with constipation, and restore their gastrointestinal motility to a normal state. At the same time, anti-inflammatory ingredients such as kaempferol, quercetin, rutin and hyperoside improve the intestinal inflammation of mice with loperamide-induced constipation and maintain the integrity of the intestinal barrier. Excess acid substances such as acetic acid and nonanoic acid are produced during the fermentation process, and these substances are beneficial to gastrointestinal digestion and absorption, and maintain the health of the intestinal ecology. The peach blossom fermentation liquid can also alleviate slow transit constipation and temporary conventional constipation caused by the opioid drugs by inhibiting the release of inhibitory gastrointestinal neurotransmitters and promoting the increase of excitatory neurotransmitters.

In summary, the peach blossom fermentation liquid provided by the present invention improves the original strong bitter taste of peach blossom and has excellent sensory quality; has high content of active ingredients, rich nutrition and strong efficacy, can play the roles of moistening intestines and relaxing bowels and promoting gastrointestinal motility, and especially has a clear and significant effect on improving constipation caused by opioid drugs.

### BRIEF DESCRIPTION OF DRAWINGS

In order to illustrate the specific embodiments of the present invention or the technical solution in the prior art more clearly, the following will briefly introduce the drawings needed to be used in the detailed description or the description of the prior art.
FIG. 1 is a flow chart of a process for preparing a peach blossom fermentation liquid in Example 1 of the present invention;
FIG. 2 is a graph showing the effects of a peach blossom liquid before and after fermentation on defecation parameters of mice with constipation in Embodiment 4 of the present invention, wherein (A) is a statistical graph of the number of red stools in 6 h, (B) is a statistical graph of the number of feces in 6 h, and (C) is a statistical graph of the water content of the feces in 6 h;
FIG. 3 is a graph showing the effects of the peach blossom liquid before and after fermentation on the time to first red stool excretion in the mice with constipation in Embodiment 4 of the present invention;
FIG. 4 is a graph showing the effects of the peach blossom liquid before and after fermentation on a gastric emptying rate in the mice with constipation in Embodiment 4 of the present invention;
FIG. 5 is a graph showing the effects of the peach blossom liquid before and after fermentation on a small intestinal propulsion rate in the mice with constipation in Embodiment 4 of the present invention;
FIG. 6 is a graph showing the effects of the peach blossom liquid before and after fermentation on excitatory gastrointestinal neurotransmitters in the mice with constipation in Embodiment 4 of the present invention, wherein (A) is a statistical graph of a serum MTL content, (B) is a statistical graph of a serum GAS content, and (C) is a statistical graph of a serum SP content; and
FIG. 7 is a graph showing the effects of the peach blossom liquid before and after fermentation on inhibitory gastrointestinal neurotransmitters in mice with constipation in Embodiment 4 of the present invention, wherein (A) is a statistical graph of a serum VIP content, (B) is a statistical graph of a serum SS content, and (C) is a statistical graph of a serum 5-HT content.

### DETAILED DESCRIPTION

The following examples are provided for better understanding of the present invention.

### Source of raw materials:

Flavourzyme (flavor enzyme): food grade, 30,000 U/g, Hebei Wanbang Chemical Technology Co., Ltd.;
cellulase: 10,000 U/g, Nanning Pangbo Bioengineering Co., Ltd.;
*Saccharomyces cerevisiae* powder: Angel Yeast Co., Ltd.;
*Lactobacillus plantarum* powder: *Lactobacillus plantarum* N13, 10 billion cfu/g, Shandong Weiang Biological Co., Ltd.;
*Lactobacillus bulgaricus* powder: 10 billion cfu/g, Shandong Weiang Biological Co., Ltd.;
*Lactobacillus helveticus* powder: 10 billion cfu/g, Shaanxi Lanhe Biotechnology Co., Ltd.;
*Leuconostoc mesenteroides subsp. mesenteroides* powder: 10 billion cfu/g, Shanghai Qiming Biotechnology Co., Ltd.;
*Streptococcus thermophilus* powder: 100 billion cfu/g, Shandong Weiang Biological Co., Ltd.

If no specific experimental steps or conditions are specified in the examples, the experiments can be carried out according to the conventional experimental steps or conditions described in the literatures in the art. All raw materials and instruments as used are commercially-available conventional products, including but not limited to the raw materials and instruments employed in the examples of the present application.

### Example 1

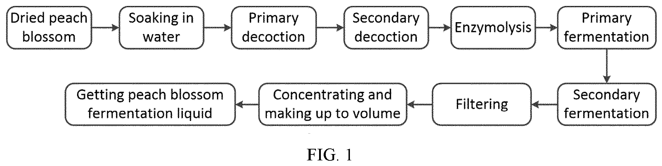

This example provided a method for preparing peach blossom fermentation liquid, the process route of which was shown in FIG. 1, and the specific steps were as follows:
(1) 100 g of dry peach blossom without stalks was weighed, added with 20 times of distilled water (based on the weight of the dry peach blossom) and soaked for 30 min, decocted at high heat (2,100 W) for 20 min, decocted at medium heat (1,400 W) for 20 min, decocted at low heat (800 W) for 20 min, and filtered. The filter residue was added with 10 times of distilled water (based on the weight of the dry peach blossom) again, decocted at high heat (2,100 W) for 40 min, and decocted at low heat (800 W) for 40 min. The filtrates and the filter residues of the two times were combined to obtain a peach blossom liquid with dregs.
(2) The peach blossom liquid with dregs obtained in the step (1) was added with flavourzyme and cellulase, and subjected to enzymolysis at 55°C for 120 min, wherein the added amount of the flavourzyme was 1.5% by mass of the peach blossom liquid with dregs, and the added amount of the cellulase was 0.75% by mass of the peach blossom liquid with dregs. After the enzymolysis was completed, the enzymes were inactivated at 110°C for 20 min to obtain an enzymolysis dreg-liquid mixture.
(3) The enzymolysis dreg-liquid mixture obtained in the step (2) was added with white granulated sugar in an amount of 5% by mass of the enzymolysis dreg-liquid mixture, sterilized at 100°C for 5 min, and cooled to room temperature to obtain a fermentation substrate.
(4) The fermentation substrate obtained in the step (3) was added with *Saccharomyces cerevisiae* powder, and subjected to a primary fermentation in a shaker, wherein the conditions for the primary fermentation were: a shaker temperature of 30 ± 2°C, a shaker rotation speed of 120 r/min, and a fermentation time of 4 d. The added amount of the *Saccharomyces cerevisiae* powder was 3% by mass of the fermentation substrate. After the fermentation was completed, it was sterilized at 85°C for 20 min, and cooled to room temperature to obtain a primary fermentation liquid.
(5) The primary fermentation liquid obtained in the step (4) was added with a composite lactic acid bacteria liquid, and subjected to a secondary fermentation in a shaker, wherein the conditions for the secondary fermentation were: a shaker temperature of 33 ± 1°C, a shaker rotation speed of 120 r/min, and a fermentation time of 3 d. The added amount of the composite lactic acid bacteria liquid was 4% by volume of the fermentation substrate. After the fermentation was completed, it was sterilized at 85°C for 20 min, cooled to room temperature, and filtered. The filtrate was concentrated and brought to the volume of 1/3 of the original volume, so as to obtain the peach blossom fermentation liquid.

The composite lactic acid bacteria liquid was obtained by activating composite lactic acid bacteria powder, and the composite lactic acid bacteria powder was obtained by mixing the following 5 kinds of bacteria powder in proportion: *Lactobacillus plantarum* N13 powder: *Lactobacillus bulgaricus* powder: *Lactobacillus helveticus* powder: *Leuconostoc mesenteroides subsp. mesenteroides* powder: *Streptococcus thermophilus* powder = 1:1.5:1:1:1.5. Activation method: the composite lactic acid bacteria powder was added into a sterilized MRS liquid medium and then cultured in a constant-temperature shaker at room temperature for 30 min to obtain the composite lactic acid bacteria liquid. The viable bacteria count of *Lactobacillus plantarum* in the obtained bacteria liquid was 1.5 × 10⁸ cfu/mL, the viable bacteria count of *Lactobacillus bulgaricus* was 3 × 10⁶ cfu/mL, the viable bacteria count of *Lactobacillus helveticus* was 1 × 10⁶ cfu/mL, the viable bacteria count of *Leuconostoc mesenteroides subsp. mesenteroides* was 1 × 10⁸ cfu/mL, and the viable bacteria count of *Streptococcus thermophilus* was 3 × 10 ⁶ cfu/mL.

### Comparative Example 1

This comparative example provided a peach blossom liquid, which was obtained by filtering the enzymatic dreg-liquid mixture obtained in the step (2) of Example 1 by suction, and concentrating the filtrate to bring to the volume of 1/3 of the original volume.

### Comparative Example 2

This comparative example provided a peach blossom fermentation liquid, and its preparation method referred to Example 1, except that: the order of the step (4) and the step (5) was interchanged, that was, the composite lactic acid bacteria liquid was first added into the fermentation substrate obtained in the step (3) for a primary fermentation, and then the *Saccharomyces cerevisiae* powder was added into the primary fermentation liquid obtained in the step (4) for a secondary fermentation, wherein the fermentation conditions and inoculation amount remained unchanged.

### Comparative Example 3

This comparative example provided a peach blossom fermentation liquid, and its preparation method referred to Comparative Example 2, except that the composite lactic acid bacteria powder was obtained by mixing *Lactobacillus bulgaricus* powder and *Streptococcus thermophilus* powder in a mass ratio of 1:1.

### Comparative Example 4

This comparative example provided a peach blossom fermentation liquid, and its preparation method referred to Example 1, except that: the primary fermentation time in the step (4) was extended to 7 d, the step (5) was omitted, the primary fermentation liquid was directly filtered, and the filtrate was concentrated and brought to the volume of 1/3 of the original volume, so as to prepare the peach blossom fermentation liquid.

### Comparative Example 5

This comparative example provided a peach blossom fermentation liquid, and its preparation method referred to Example 1, except that: the step (4) was omitted, and the composite lactic acid bacteria liquid was directly added into the fermentation substrate obtained in the step (3) for fermentation, and the fermentation time was extended to 7 d.

### Comparative Example 6

This comparative example provided a peach blossom fermentation liquid, and its preparation method referred to Example 1, except that: the *Saccharomyces cerevisiae* powder and the composite lactic acid bacteria liquid were added into the fermentation substrate obtained in the step (3) for co-fermentation, and the conditions for the fermentation were: a shaker temperature of 30 ± 3°C, a shaker rotation speed of 120 r/mim, and a fermentation time of 7 d. The added amount of the *Saccharomyces cerevisiae* powder was 3% by mass of the fermentation substrate, and the added amount of the composite lactic acid bacteria liquid was 2% by volume of the fermentation substrate.

### Comparative Example 7

This comparative example provided a peach blossom fermentation liquid, and its preparation method referred to Example 1, except that: in the step (5), the composite lactic acid bacteria powder was obtained by mixing the following 3 kinds of bacteria powder in proportion: *Lactobacillus plantarum* N13 powder: *Lactobacillus bulgaricus* powder: *Thermophilic Streptococcus* powder = 1:1.5:1.5.

### Comparative Example 8

This comparative example provided a peach blossom fermentation liquid, and its preparation method referred to Example 1, except that: in the step (5), the composite lactic acid bacteria powder was obtained by mixing the following 3 kinds of bacteria powder in proportion: *Lactobacillus bulgaricus* powder: *Lactobacillus helveticus* powder: *Thermophilic Streptococcus* powder = 1.5:1:1.5.

### Comparative Example 9

This comparative example provided a peach blossom fermentation liquid, and its preparation method referred to Example 1, except that: in the step (5), the composite lactic acid bacteria powder was obtained by mixing the following 3 kinds of bacteria powder in proportion: *Lactobacillus plantarum* N13 powder: *Lactobacillus helveticus* powder: *Leuconostoc mesenteroides subsp. mesenteroides* powder = 1:1:1.

### Comparative Example 10

This comparative example provided a peach blossom fermentation liquid, and its preparation method referred to Example 1, except that: in the step (5), the composite lactic acid bacteria powder was obtained by mixing the following 4 kinds of bacteria powder in proportion: *Lactobacillus plantarum* N13 powder: *Lactobacillus bulgaricus* powder: *Lactobacillus helveticus* powder: Streptococcus thermophilus powder = 1:1.5:1:1.5.

### Comparative Example 11

This comparative example provided a peach blossom fermentation liquid, and its preparation method referred to Example 1, except that: in the step (5), the composite lactic acid bacteria powder was obtained by mixing the following 4 kinds of bacteria powder in proportion: *Lactobacillus plantarum* N13 powder: *Lactobacillus bulgaricus* powder: *Leuconostoc mesenteroides subsp. mesenteroides* powder: Streptococcus thermophilus powder = 1:1.5:1:1.5.

### Comparative Example 12

This comparative example provided a peach blossom fermentation liquid, and its preparation method referred to Example 1, except that: in the step (5), the composite lactic acid bacteria powder was obtained by mixing the following 4 kinds of bacteria powder in proportion: *Lactobacillus bulgaricus* powder: *Lactobacillus helveticus* powder: *Leuconostoc mesenteroides subsp. mesenteroides* powder: Streptococcus thermophilus powder = 1.5:1:1:1.5.

### Embodiment 1 Determination of active ingredients

### 1. Determination of chemical ingredient contents in Example 1 and Comparative Examples 1-2 and 4-6

Total sugar and polysaccharide content: They were determined by a phenol-sulfuric acid method, and a standard curve was plotted with a glucose standard solution as a horizontal axis and the absorbance as a vertical axis. The resultant standard curve equation was: y = 0.0115x + 0.0088, R² = 0.9987.

Total phenol content: It was determined by a Folin phenol method, and a standard curve was plotted with the concentration of a gallic acid standard solution as the horizontal axis and the absorbance as the vertical axis. The resultant standard curve equation was: y = 0.0207x - 0.004, R² = 0.9932.

Total flavonoid content: the total flavonoid content in the peach blossom liquid and each peach blossom fermentation liquid were determined by the phenol-sulfuric acid method. A standard curve of concentration was plotted with the concentration of a rutin standard solution as the horizontal axis and the absorbance as the vertical axis. The resultant standard curve equation was: y = 0.0023x - 8E⁻⁰⁵, R² = 0.9997.

Protein Content: it was determined according to a kjeldahl nitrogen determination method of GB5009.5-2016.

Dietary fiber content: it was determined according to an enzyme weight method of GB5009.88-2014.

Total acid content: it was determined according to an acid-base indicator titration method of GB12456-2021.

The determination results of respective ingredients were shown in Table 1.

**Table 1 Determination Results of Contents of Chemical Ingredients**

| Ingredients (g/100 g) | Example 1 | Comparative Example 1 | Comparative Example 2 | Comparative Example 4 | Comparative Example 5 | Comparative Example 6 |
|---|---|---|---|---|---|---|
| Total Sugar | 25.310±0.021 | 17.337±0.015 | 20.245±0.061 | 19.628±0.314 | 23.381±0.056 | 21.110±0.010 |
| Polysac charide | 8.838±0.105 | 7.427±0.067 | 8.567±0.021 | 4.494±0.031 | 2.695±0.09 | 5.564±0.251 |
| Total phenol | 25.276±0.098 | 8.433±0.134 | 9.379±0.245 | 10.056±0.389 | 6.804±0.008 | 16.996±0.156 |
| Total flavonoid | 4.090±0.261 | 5.295±0.149 | 4.295±0.063 | 4.626±0.028 | 5.629±0.057 | 3.675±0.162 |
| Protein | 16.875±0.089 | 26.541±0.216 | 6.091±0.514 | 7.133±0.105 | 6.343±0.114 | 5.875±0.089 |
| Dietary fiber | 29.050±0250 | 14.750±0.650 | 10.600±0.300 | 8.750±0.650 | 6.850±1.050 | 15.050±0.750 |
| Total acid | 4.420±0.063 | 1.356±0.089 | 3.145±0.025 | 2.985±0.075 | 3.385±0.035 | 2.885±0.034 |

As shown in Table 1, the peach blossom fermentation liquid prepared in Example 1 had higher contents of total sugar, polysaccharide, total phenol, dietary fiber and total acid than those of the other comparative examples, especially the increase of the polysaccharide, total phenol, dietary fiber and total acid was particularly obvious, which proved that the manner of first fermenting with *Saccharomyces cerevisiae* and then fermenting with the composite lactic acid bacteria had obvious advantages in increasing the content of active ingredients compared with no fermentation, single type of fermentation and simultaneous one-step fermentation. The increase in the total sugar, the total phenol and the dietary fiber content was beneficial to promoting intestinal peristalsis and maintaining a healthy intestinal environment.

### 2. Determination of contents of phenolic and flavonoid substances in Example 1 and Comparative Example 1

The contents of chlorogenic acid, rutin, hyperoside, quercetin, naringenin, ferulic acid and kaempferol in the samples were determined by a HPLC method. The chromatographic conditions were: Amnex HPX-87H column: acetonitrile was used as mobile phase A and a 0.1% acetic acid aqueous solution was used as mobile phase B. Gradient elution was performed according to the elution procedure in Table 2; the flow rate was 0.8 mL/min, the column temperature was 30°C, and the injection volume was 10 µL, wherein the detection wavelength of chlorogenic acid was 320 nm, the detection wavelength of rutin, hyperoside, quercetin and kaempferol was 254 nm, and the detection wavelength of ferulic acid and naringenin was 320 nm. Chlorogenic acid, kaempferol, quercetin, rutin, naringenin, and kaempferol and ferulic acid were used as reference substances, and quantitatively and qualitatively determined according to the peak areas and retention times. The contents of the substances were calculated by comparing with the standard working curve. The determination results were shown in Table 3.

**Table 2 Mobile-phase gradient elution sequence**

| Time (min) | Mobile phase A (%) | Mobile phase B (%) |
|---|---|---|
| 0-5 | 20 | 80 |
| 5-25 | 60 | 40 |
| 25-28 | 95 | 5 |
| 28-30 | 5 | 95 |
| 30-36 | 5 | 95 |

**Table 3 Determination results of phenolic and flavonoid ingredients**

| Ingredient content (µg/g) | Standard curve equation | R² | Comparative Example 1 | Example 1 |
|---|---|---|---|---|
| Chlorogenic acid | y=27.121x + 108.78 | R² = 0.9975 | 13.065 | 31.642 |
| Kaempferol | y= 21.686x - 6.8219 | R² = 0.9999 | 0.205 | 0.393 |
| Quercetin | y=17.563x-18.166 | R² = 0.9995 | 3.477 | 4.673 |
| Ferulic acid | y=61.056x + 1.2441 | R² = 1 | 1.084 | 0.550 |
| Rutin | y=21.263x-6.9991 | R² = 0.9998 | 1.417 | 1.771 |
| Naringenin | y = 9.504x + 1.5058 | R² = 0.9999 | 2.034 | 1.025 |
| Hyperoside | y= 15.466x - 1.7676 | R² = 0.9999 | 3.477 | 4.673 |

As shown in Table 3, compared with the unfermented peach blossom liquid prepared in Comparative Example 1, the peach blossom fermentation liquid prepared in Example 1 had increased contents of chlorogenic acid, kaempferol, quercetin, rutin and hyperoside, which was beneficial to improving intestinal inflammation and maintaining the integrity of the intestinal barrier.

### Embodiment 2 Sensory Evaluation

A sensory evaluation team was composed of 30 experienced personnel (15 males and 15 females) screened by food sensory analysis. According to the sensory scoring criteria provided in Table 4, they made sensory evaluation on the peach blossom fermentation liquid prepared in Example 1 and Comparative Examples 1-12, and the results were shown in Table 5.

**Table 4 Sensory scoring criteria**

| Scoring items | Criteria | Score (points) |
|---|---|---|
| Aroma (30 points) | Rich fruity and fermented aroma, no odor | 20-30 |
| | Light fruity and fermented aroma, with a slight odor | 10-19 |
| | No fruity or fermented aroma, with unpleasant odor | 0-9 |
| Taste (30 points) | Balanced sweet and sour taste, rich taste, and no sour and bitter taste | 20-30 |
| | Poor taste, slightly sour or slightly sweet, with a bitter taste | 10-19 |
| | The taste is not harmonious, the sweet and sour taste is unbalanced, and the bitter taste is strong | 0-9 |
| Color and lustre (20 points) | Harmonious color and lustre, and good gloss | 14-20 |
| | There is a deviation in the color and lustre, with a little bit of mottled color in some parts | 7-13 |
| | Unharmonious colour and lustre, obvious local mottled color, and poor gloss | 0-6 |
| Status (20 points) | Uniform texture, clear, with no suspended matter | 14-20 |
| | Slightly turbid and containing a precipitate | 7-13 |
| | Obvious stratification, turbidity, with more suspended matters | 0-6 |

**Table 5 Sensory evaluation results**

| Groups | Aroma (30 points) | Taste (30 points) | Colour and lustre (20 points) | State (20 points) | Total score (100 points) |
|---|---|---|---|---|---|
| Example 1 | 23.25±0.507 | 19.73±0.640 | 23.45±0.387 | 22.13±0.330 | 88.80±0.383 |
| Comparative Example 1 | 12.50±0.577 | 4.50±0.577 | 15.50±1.291 | 22.00±0.816 | 52.50±1.915 |
| Comparative Example 2 | 22.70±0.668 | 19.23±0.685 | 22.90±0.753 | 20.88±0.854 | 85.70±1.378 |
| Comparative Example 3 | 22.43±0.995 | 18.15±0.545 | 22.53±0.892 | 21.10±0.316 | 84.20±1.977 |
| Comparative Example 4 | 9.50±0.577 | 10.50±0.577 | 17.50±1.291 | 23.75±0.957 | 61.25±0.957 |
| Comparative Example 5 | 21.00±0.816 | 12.75±0.500 | 20.75±0.957 | 15.00±1.414 | 69.50±1.291 |
| Comparative Example 6 | 22.60±0.346 | 19.85±0.387 | 22.78±0.714 | 20.95±0.835 | 86.175±0.492 |
| Comparative Example 7 | 21.48±0.709 | 21.28±1.226 | 19.83±0.624 | 20.58±0.68 | 82.90±1.359 |
| Comparative Example 8 | 21.75±0.645 | 20.85±0.755 | 20.08±0.718 | 20.28±0.78 | 82.95±0.850 |
| Comparative Example 9 | 21.65±0.480 | 19.88±0.922 | 18.75±0.854 | 20.20±0.678 | 80.48±1.513 |
| Comparative Example 10 | 21.88±0.299 | 18.93±0.359 | 21.63±0.506 | 20.18±0.826 | 82.55±0.759 |
| Comparative Example 11 | 22.45±0.493 | 19.08±0.562 | 22.55±0.480 | 20.55±0.465 | 84.425±0.718 |
| Comparative Example 12 | 20.85±0.532 | 19.53±0.275 | 22.80±0.440 | 20.88±0.768 | 84.06±0.866 |

As shown in Table 5, compared with the products obtained from other different strains and different fermentation manners, the peach blossom fermentation liquid prepared in Example 1 had improved sensory scores, and better comprehensive sensory evaluation. After the primary fermentation with *Saccharomyces cerevisiae* and the composite secondary fermentation with 5 kinds of lactic acid bacteria, the fermentation time was shortened, the neutralization of acid and alcohol was better, the content of aroma ingredients such as ester substances was increased, the texture of the peach blossom liquid was more uniform, and the solution was clearer and more transparent. However, the peach blossom fermentation liquid obtained by the simultaneous fermentation with *Saccharomyces cerevisiae* and the 5 kinds of lactic acid bacteria had a higher content of produced alcohol substances, and had basically no excess acid substances as produced, the bitter almond aroma of it was still strong, and the taste of it was poor. Comparative Examples 7-12 employed 3 or 4 lactic acid bacteria for composite fermentation, and their sensory evaluation results were obviously not as good as those of Example 1, which proved that the composite fermentation of 5 specific lactic acid bacteria provided by the present invention had obvious advantages in improving the sensory properties of the peach blossom fermentation liquid.

### Embodiment 3 Determination of volatile substances

The volatile substances in the peach blossom fermentation liquid prepared in Example 1 and Comparative Example 6 and the peach blossom liquid prepared in Comparative Example 1 were determined by gas chromatography-mass spectrometry (GC-MS).

The determination method was as follows: 6 mL of a sample to be tested was taken and placed in a 20 mL top empty bottle, added with 5 µL of 2-methyl 3-heptanone internal standard at a concentration of 0.5 µg/mL, and mixed well by shaking gently. Then the top empty injection bottle was covered with a cap, and then equilibrated in a water bath at a condition of 50°C for 10 min. A 50/30 µm DVB/CAR/PDMS extraction head was inserted into the top empty bottle for adsorption for 30 min, and a fiber head was inserted into the gas chromatography system for desorption for 3 min at a desorption temperature of 250°C.

Chromatographic conditions: the capillary column was DB-WAX ( 30 m×0.25 mm, 0.25 µm); the injection volume was 2 µL; inlet temperature was 250°C; and the heating procedure was: maintaining at an initial column temperature of 40°C for 3 min, then increasing to 240°C at a rate of 5°C/min and maintaining at this temperature for 10 min. The flow rate of the carrier gas (He) was 1.0 mL/min.

Mass spectrometry conditions: a ion source temperature of 230°C, and a quadrupole temperature of 150°C; an electron impact (EI) ion source; a mass spectrometer interface temperature of 250°C; electron energy of 70 eV; and ionization mode EI: mass scan range (m/z 33-550).

The determination results were shown in Table 6.

**Table 6 Determination Results of Volatile Substances**

| Compound Name | Retention time (min) | Relative content (%) | | | Odor characteristics |
|---|---|---|---|---|---|
| | | Comparative Example 1 | Example 1 | ComparativeExample 6 | |
| Esters | | | | | |
| ethyl 2-methyl-butyrate | 5.893 | - | 1.38 | 1.06 | Fruity, and ester aroma |
| Ethyl butyrate | 5.535 | - | 8.09 | - | Sweet fruity aroma |
| Ethyl valerate | 7.899 | 4.25 | 10.89 | - | Apple aroma, peach aroma, and grape aroma |
| Ethyl 4-enoate | 12.392 | 0.12 | - | - | |
| Ethyl hexanoate | 10.76 | 33.57 | - | - | Yeast aroma, pineapple aroma |
| Ethyl 3-hexenoate | 12.647 | 6.38 | 17.44 | - | fruity fresh scent |
| Hexyl formate | 14.215 | - | 0.25 | - | Jackfruit aroma |
| Ethyl caprylate | 16.291 | 0.37 | - | - | Apricot aroma |
| Methyl benzoate | 20.863 | - | 0.18 | - | Faint fresh scent |
| Ethyl benzoate | 21.934 | 0.79 | 3.65 | 1.87 | Fruity aroma |
| Ethyl 3-hydroxy-hexanoate | 22.333 | - | 0.48 | - | Fruity aroma |
| Benzyl acetate | 23.356 | - | 0.47 | 0.83 | Jasmine aroma |
| Methyl salicylate | 24.366 | - | - | 0.29 | Holly aroma |
| Ethyl oleate | 37.917 | 0.08 | - | - | Fresh flower aroma |
| Ethyl palmitate | 34.069 | - | - | 0.30 | Butter aroma, wax aroma |
| Aldehydes | | | | | |
| 3-methyl-hexanal | 16.916 | 0.12 | - | - | |
| Benzaldehyde | 18.393 | 30.73 | 14.28 | 20.27 | Bitter almond aroma |
| 5-methyl-furfural | 19.67 | - | - | 0.08 | sweet, and spicy aroma |
| 2-hydroxybenzaldehyde | 22.225 | - | 0.11 | - | |
| 2,4-dimethylbenzaldehyde | 25.151 | 1.11 | - | - | Bitter almond aroma |
| Alcohols | | | | | |
| Ethanol | 4. 02 | - | 3. 21 | 0. 75 | Wine aroma |
| Pentanol | 11 .458 | - | 0. 13 | - | Fusel-like special aroma |
| Hexanol | 14 .244 | - | 0. 60 | 0. 77 | Grassy aroma |
| 2-ethyl-1-hexanol | 17 .795 | 0. 19 | - | - | Pungent smell |
| Linalool | 19 .216 | - | - | 2. 06 | Lily of the valley aroma |
| Octanol | 19 .504 | - | 0. 52 | 0. 81 | Greasy aroma, and citrusy aroma |
| Nonanol | 21.946 | - | - | 1.73 | Orange aroma |
| 3-nonenol | 22 .477 | - | 0. 25 | 0. 44 | Passion fruit aroma |
| Terpineol | 22 .757 | - | 0. 29 | 0. 27 | Pittosporum flower fresh scent, lily of the valley aroma |
| 1-Nonanol | 26 .088 | - | 0. 44 | 0. 34 | Rose scent |
| Benzyl alcohol | 26 .208 | 2. 76 | 2 6.73 | 1 9.73 | Faint aroma |
| Phenylethyl alcohol | 27 .319 | 1. 62 | 1. 65 | 1. 05 | Flower scent |
| Oleyl alcohol | 28 .333 | - | - | 0. 10 | Cool herbal flavor |
| 3-phenylpropanol | 30 .035 | 0. 13 | 0. 32 | 0. 35 | Fruity, honey, and floral aroma |
| Acids | | | | | |
| Glutaric acid | 13 .95 | 0. 17 | - | - | |
| Hexanoic acid | 14 .075 | 0. 17 | - | - | |
| Acetic acid | 16 .773 | - | 2. 56 | 1. 55 | Vinegar flavor |
| Octanoic acid | 32 .812 | - | 0. 17 | - | Fruity aroma |
| Nonanoic acid | 33 .363 | - | 4. 06 | - | Coconut aroma |
| Phenylacetic acid | 34 .545 | - | 0. 26 | - | Honey aroma |
| n-decanoic acid | 35 .795 | - | 0. 31 | - | Juice aroma |
| Benzoic acid | 40 .995 | - | 0. 12 | - | |
| Ketones | | | | | |
| Acetophenone | 12 .181 | 0. 51 | 0. 82 | 1. 01 | Orange aroma |
| 4-hydroxy-2-methylacetophenone | 32 .812 | 0. 52 | | 2. 27 | Red wine aroma |
| 4-hydroxy-3-methylacetophenone | 32.813 | - | 1.45 | - | |
| 2-hendecanone | 20 .411 | | 0. 15 | 1. 03 | Coconut, rose, and citrus aroma |
| 2,3-dihydro-3,5-dihydroxy-6-methyl-4H-pyran-4-one | 34 .147 | 0. 19 | - | - | Caramel-like aroma |
| Phenols | | | | | |
| 4-ethyl-2-methoxy-phenol | 29 .689 | | 0. 80 | 0. 23 | Sweet and warm spicy flavor, and herbal aroma |
| 3-allyl-6-methoxyphenol | 32 .304 | | 0. 59 | 0. 11 | Clove's spicy aroma |
| 4-ethyl-phenol | 32 .454 | | 1 7.47 | 1 2.99 | Strong wood phenol smell, and slightly sweet aroma |

It could can be seen from Table 6 that, compared with Comparative Example 1, in Example 1 the contents of substances with bitter almond aroma such as 2,4-dimethylbenzaldehyde and benzaldehyde in the peach blossom fermentation liquid were reduced, and the contents of main flavor substances such as ethyl butyrate, ethyl valerate, ethyl 3-hexenoate, ethyl benzoate, ethyl 3-hydroxy-hexanoate, benzyl alcohol, acetic acid, nonanoic acid, acetophenone, ethyl valerate, ethyl 3-hexenoate, and 4-ethyl-phenol were increased, while in Comparative Example 6, in addition to flavor substances such as benzaldehyde, benzyl alcohol, ethyl benzoate, and 4-ethyl-phenol, some alcohol substances such as linalool, octanol, and nonanol were also produced, but no excess acid substance was produced. It was proved that the preparation method provided in Example 1 of the present invention was more conducive to obtaining a peach blossom fermentation liquid with excellent flavor.

### Embodiment 4 Animal Experiment

### 1. Experimental Animals

Male SPF-gade ICR mice (20 ± 2 g) were purchased from Guangdong Charles River Laboratory Animal Technologies Co. Ltd., with license number: SYXK (Yue)2022-0136, kept in a SPF-grade barrier environment of the Experimental Animal Center of South China Agricultural University and were raised according to the experimental animal protection and ethical rules. The room temperature was controlled at 20-26°C, the humidity was controlled in the range of 40-70%, the light and dark were alternated for 12 h each, ventilation was ensured, and the mice had free access to feedstuff and drinking water during the period.

### 2. Experimental Grouping and Treatment

After acclimating to standard conditions for 7 days, the mice were randomly divided into 7 groups with 8 mice in each group, and subjected to gavage according to Table 7.

**Table 7 Animal experiment design**

| Grouping | Number of animals | Gavage volume | First 5 days | | Day 6-12 | |
|---|---|---|---|---|---|---|
| | | | Morning | Afternoon | Morning | Afternoon |
| Blank control group | 8 | 0.2 mL/10 g | Sterile water | | Sterile water | Sterile water |
| Constipation model group 1 | 8 | 0.2 mL/10 g | Loperamide hydrochloride (10 mg/kg) | | Loperamide hydrochloride (10 mg/kg) | Sterile water |
| Experimental Group 1 | 8 | 0.2 mL/10 g | Loperamide hydrochloride (10 mg/kg) | | Loperamide hydrochloride (10 mg/kg) | Peach blossom fermentation liquid prepared in Example 1 (1 g/kg) |
| Experimental Group 2 | 8 | 0.2 mL/10 g | Loperamide hydrochloride (10 mg/kg) | | Loperamide hydrochloride (10 mg/kg) | Peach blossom liquid prepared in Comparative Example 1 (1 g/kg) |
| Experimental Group 3 | 8 | 0.2 mL/10 g | Loperamide hydrochloride (10 mg/kg) | | Loperamide hydrochloride (10 mg/kg) | Peach blossom fermentation liquid prepared in Comparative Example 6 (1 g/kg) |
| Positive control group 1 | 8 | 0.2 mL/10 g | Loperamide hydrochloride (10 mg/kg) | | Loperamide hydrochloride (10 mg/kg) | Mosapride (20 mg/kg) |
| Constipation model group 2 | 8 | 0.2 mL/10 g | Fed with rice + limited water | | Fed with rice + limited water | Sterile water |
| Experimental Group 4 | 8 | 0.2 mL/10 g | Fed with rice + limited water | | Fed with rice + limited water | Peach blossom fermentation liquid prepared in Example 1 (1 g/kg) |
| Experimental Group 5 | 8 | 0.2 mL/10 g | Fed with rice + limited water | | Fed with rice + limited water | Peach blossom liquid prepared in Comparative Example 1 (1 g/kg) |
| Experimental Group 6 | 8 | 0.2 mL/10 g | Fed with rice + limited water | | Fed with rice + limited water | Peach blossom fermentation liquid prepared in Comparative Example 6 (1 g/kg) |
| Positive control group 2 | 8 | 0.2 mL/10 g | Fed with rice + limited water | | Fed with rice + limited water | Mosapride (20 mg/kg) |

### 3. Pharmaceutical effect evaluation

After 8 days of continuous intervention, the time to the first red stool excretion, the number of red stools at 6 h, the number of stools and the water content of stools at 6 h were observed to evaluate the effect of moistening intestines and relaxing bowels. After 9 days of continuous intervention, the gastric emptying rate, small intestine propulsion rate and the expression level of gastrointestinal neurotransmitters in serum were determined anatomically to evaluate their effects on gastrointestinal motility in the mice.
(1) Determination of defecation parameters: After 8 days of continuous intervention, the mice in each group fasted for 12 h without stopping drinking water, and each mouse was subjected to gavage of 0.3 mL of a magenta solution, and the time of gavage was recorded. After the gavage, the mice were placed in a clean cage lined with filter paper and ate and drank normally. The time to the first red stool excreted by each mouse was observed and recorded. The number of red stools and the total number of feces excreted by each mouse at 6 h were recorded, and at the same time, the fresh red stools and feces excreted at 6 h were collected into a clean centrifuge tube. The wet weight of the feces was weighed, and the feces were placed in an oven at 60°C for continuous drying for 48 h, then the feces were weighed again, and the difference between the two weights was calculated and the water content of the feces was calculated according to the following equation: $\begin{matrix}water content of feces = \left(wet weight of feces-dry weight of feces\right) / wet weight of feces \times \\ 100 %\end{matrix}$
(2) Determination of gastric emptying rate: The whole stomach of the mouse was cut off, washed with physiological saline, dried with filter paper, and then cut open along the greater curvature side of the stomach. The stomach content was washed with distilled water into a 50 mL centrifuge tube containing 10 mL distilled water, added with 20 mL of NaOH (0.5 mol/L) and stirred until uniform. The mixture was allowed to stand at room temperature for 60 min. 5 mL of the supernatant was taken, added with 0.5 mL of a trichloroacetic acid solution (20%, w/v), and centrifuged at a rotation speed of 3,500 rpm/min for 10 min. Then the supernatant was taken and determined for the absorbance at 560 nm (A_{determined phenol red}). Another 0.3 mL of a methylcellulose-phenol red solution was taken into a beaker, sequentially added with 10 mL of distilled water, 20 mL of a NaOH solution (0.5 mol/L), and 3 mL of a trichloroacetic acid solution (20%, w/v) and stirred uniformly to formulate a standard solution. The standard solution was detected for the absorbance at a wavelength of 560 nm (A_{standard phenol red}). The gastric emptying rate was calculated according to the following equation: $gastric emptying rate = \left(1-{A}_{determined phenol red}/{A}_{standard phenol red}\right) \times 100 %$
(3) Determination of small intestinal propulsion rate: After the drug intervention was completed, the mice in each group were fasted without stopping drinking water for 12 h. On the next day, each mouse was subjected to gavage of 0.3 mL of a 0.05% phenol red solution. After 30 min, the mice were anesthetized by intraperitoneal injection with 5% hydrochloric acid hydrate, subjected to blood sampling by eyeball extirpating, and then sacrificed by cervical dislocation. The mice was quickly laparotomized to take out whole small intestine. The small intestine was straightened on blank A4 paper and the total length of the small intestine and the advanced length of phenol red were measured. The small intestinal propulsion rate was calculated according to the following equation: $\begin{matrix}small intestinal propulsion rate \left(%\right) = phenol red propulsion length \left(cm\right) / total length of small \\ intestinal \left(cm\right) \times 100 %\end{matrix}$
(4) Determination of gastrointestinal neurotransmitters in serum: A Elisa kit was employed to determine the contents of SP, MTL, GAS, VIP, SS and 5-HT in the serum of the mice in each group.

### 4. Data statistics and analysis processing

Excel 2022 and GraphPad Prism 8 software were employed for plotting, and SPSS 18.0 software was employed for one-way analysis of variance of the data. p < 0.05 indicated a significant difference, and *p* < 0.01 indicated an extremely significant difference.

### 5. Results analysis

### (1) Defecation parameters

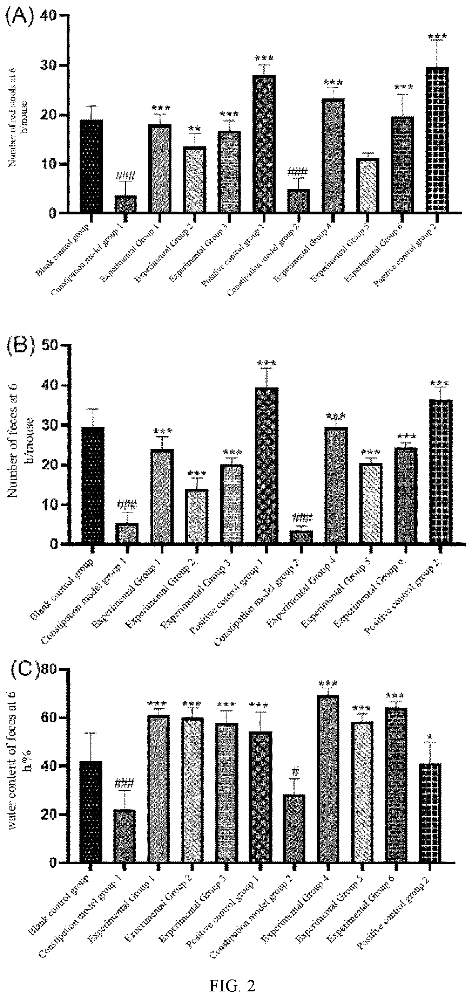

Defecation parameters were important indicators of constipation. When defecation frequency was reduced, the defecation was difficult. Reduced water content in the feces was the main symptom of constipation. Therefore, the effects of the peach blossom liquid in promoting defecation before and after fermentation were observed by measuring the defecation parameters. The results were shown in FIG. 2. Compared with the blank control group, the number of red stools and the number of feces at 6 h of the mice in the constipation model group 1 and the constipation model group 2 were significantly reduced, and the water content of feces at 6 h was significantly reduced (p < 0.05); the water content of feces at 6 h, the number of red stools and the number of feces at 6 h were significantly increased after intervention with peach blossom liquid before and after fermentation (experimental groups 1-6) *(p* < 0.05), among which the peach blossom fermentation liquid prepared in Example 1 (experimental groups 1 and 4) had a better effect in promoting defecation.

### (2) Gastric emptying rate and small intestinal propulsion rate

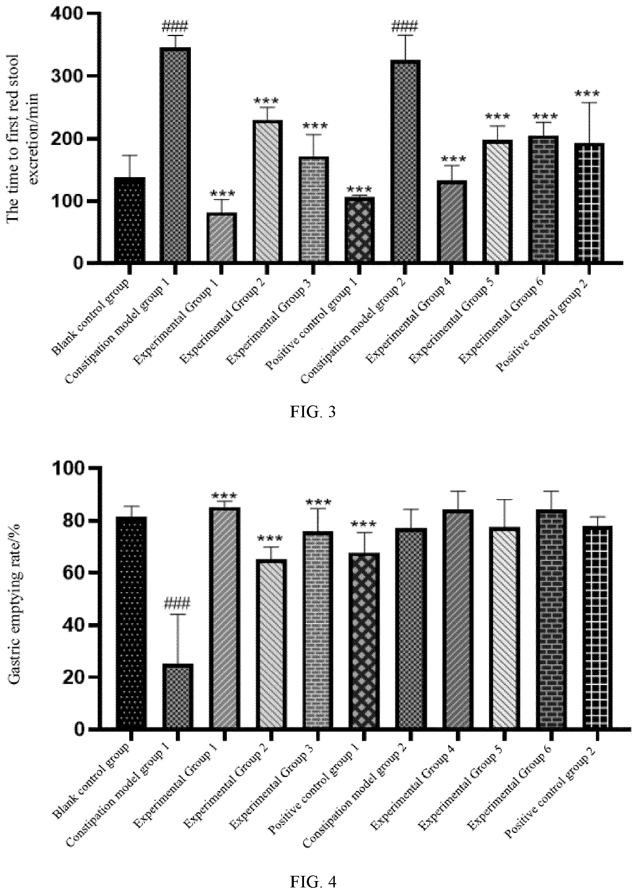

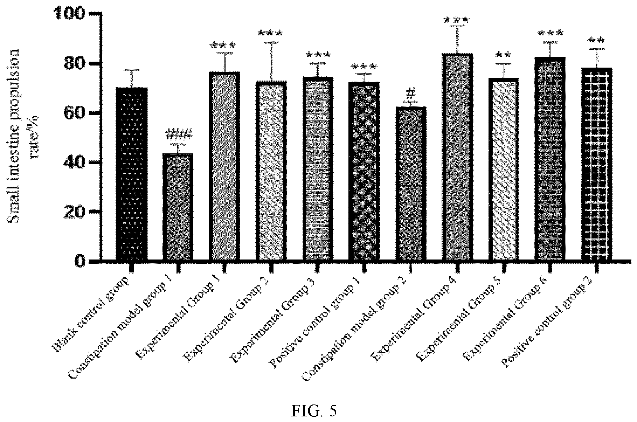

Constipation was related to gastrointestinal motility. Constipation was often manifested as reduced gastrointestinal motility and prolonged defecation time. The results of the effects of the peach blossom liquid before and after fermentation on the gastrointestinal motility in mice with constipation were shown in FIGs. 3-5. Compared with the blank control group, the mice in the constipation model group 1 had a significantly shortened time to the first red stool excretion and a significantly reduced small intestinal propulsion rate (p < 0.05); compared with the constipation model group 1, the times to the first red stool excretion and the small intestinal propulsion rates in the peach blossom liquid group (experimental group 2) and the peach blossom fermentation liquid group (experimental groups 1 and 3) were significantly increased (p < 0.05); while there was no significant difference in gastric emptying rate among the constipation model group 2, the peach blossom liquid group (experimental group 5) and the peach blossom fermentation liquid group (experimental groups 4 and 6), indicating that the method of feeding rice + limited water did not lead to reduced gastric emptying rate of the mice, while the peach blossom liquid before and after fermentation could both significantly shorten the time to the first red stool excretion of the mice with slow transit constipation caused by opioid drugs, promote gastrointestinal motility of the mice, improve gastrointestinal motility deficiency caused by constipation, and relieve constipation. Among them, the peach blossom fermentation liquid prepared in Example 1 (experimental group 1) had a better effect in promoting gastrointestinal motility.

### (3) Gastrointestinal neurotransmitters

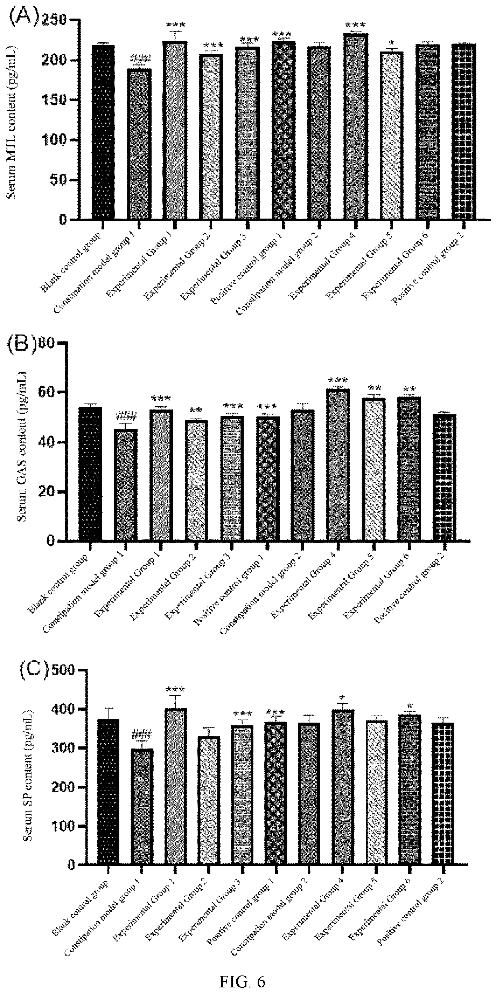

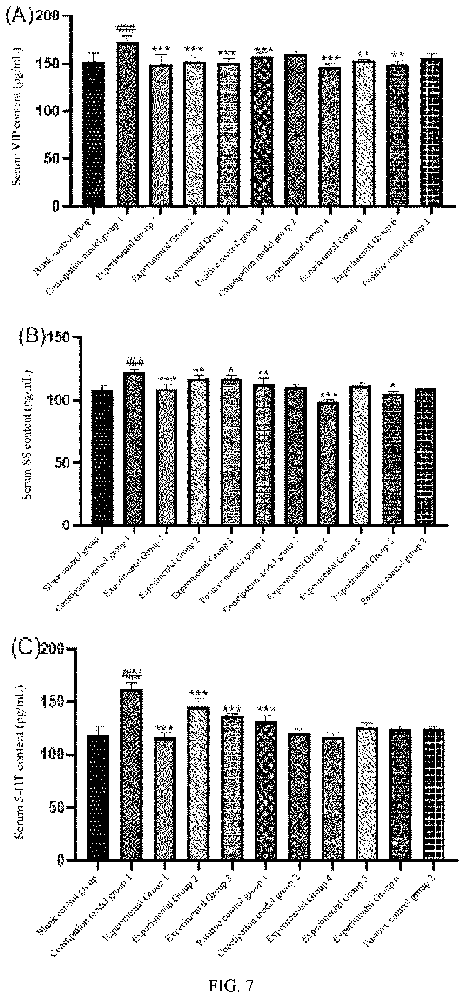

Constipation led to reduced gastrointestinal motility, and the gastrointestinal motility was closely related to gastrointestinal neurotransmitters. When excitatory gastrointestinal neurotransmitters (MTL, GAS and SP) were decreased, and inhibitory gastrointestinal neurotransmitters (VIP, SS and 5-HT) were increased, constipation worsened. Therefore, in order to further evaluate the effect of the peach blossom fermentation liquid on the gastrointestinal neurotransmitters, the expression levels of the excitatory gastrointestinal neurotransmitters (MTL, GAS and SP) and the inhibitory gastrointestinal neurotransmitters (VIP, SS and 5-HT) in mouse serum were determined. The results were shown in FIGs. 6 and 7. In the serum of the mice in the constipation model group 1, the expression levels of the excitatory neurotransmitters (MTL, GAS, SP) were significantly decreased (p < 0.05), and the expression levels of the inhibitory neurotransmitters (VIP, SS, 5-HT) were significantly increased (p < 0.05). Compared with mice in the blank control group, there was no significant difference in gastrointestinal neurotransmitters in the serum of the mice in the constipation model group 2. After gavage with the peach blossom liquid and peach blossom fermentation liquid, respectively, the expression levels of MTL, GAS, and SP in the serum of the mice in each treatment group were significantly increased (p < 0.05), and the expression levels of VIP and 5-HT were decreased (p < 0.05). Among them, the peach blossom fermentation liquid prepared in Example 1 had a better regulatory effect. It indicated that the peach blossom fermentation liquid could relieve the slow transit constipation and temporary conventional constipation induced by opioid drugs by inhibiting the release of the inhibitory gastrointestinal neurotransmitters and promoting the increase of the excitatory neurotransmitters.

### (4) Conclusion

In this experiment, constipation mouse models (slow transit constipation and temporary conventional constipation) were constructed by methods of gavage of loperamide and feeding rice + limited water, respectively, and the mice in the two constipation models were intervened with mosapride, the peach blossom fermentation liquid prepared in Example 1, the peach blossom liquid prepared in Comparative Example 1, and the peach blossom fermentation liquid prepared in Comparative Example 6. The results showed that peach blossom fermentation liquid could significantly increase the expression levels of gastrointestinal neurotransmitters in the serum of the mice with slow transit constipation induced by opioid drugs, significantly shorten the time to the first red stool excretion in the mice with constipation (p < 0.05), accelerate gastrointestinal motility, promote the defecation volume at 6 h, increase the water content of feces, soften feces, and thus relieve constipation. The gastric emptying rate and gastrointestinal neurotransmitters in the serum of the mice with constipation in the constipation model constructed by feeding rice + limited water were not significantly different from those of the blank control group, while the expression levels of the gastrointestinal neurotransmitters in the mice gavaged with the peach blossom fermentation liquid prepared in Example 1 and Comparative Example 6 were significantly different from those of the mice in the constipation model, indicating that the main symptoms of constipation in the mice with constipation constructed by feeding rice + limited water were mainly manifested in changes in defecation, and this type of constipation was milder than the constipation symptoms of the mice with constipation induced by loperamide. It could be inferred that the peach blossom fermentation liquid provided by the present invention could improve both the temporary conventional constipation and the slow transit constipation caused by opioid drugs, and could be used for preparing foods, health care products or medicines for improving constipation.

## Claims

1. A method for preparing a peach blossom fermentation liquid, comprising the following steps:
(1) soaking dried peach blossoms in water and then decocting to obtain a peach blossom liquid with dregs;
(2) adding a flavourzyme and a cellulase to the peach blossom liquid with dregs for enzymolysis, and inactivating the enzymes to obtain an enzymolysis dreg-liquid mixture;
(3) adding a carbon source into the enzymolysis dreg-liquid mixture, and sterilizing to obtain a fermentation substrate;
(4) inoculating *Saccharomyces cerevisiae* into the fermentation substrate for a primary fermentation, and sterilizing after the fermentation is completed to obtain a primary fermentation liquid; and
(5) inoculating composite lactic acid bacteria into the primary fermentation liquid for a secondary fermentation, sterilizing after the fermentation is completed, filtering, and concentrating to obtain the peach blossom fermentation liquid, wherein the composite lactic acid bacteria comprise *Lactobacillus plantarum, Lactobacillus bulgaricus, Lactobacillus helveticus, Leuconostoc mesenteroides subsp. mesenteroides,* and *Streptococcus thermophilus.*

2. The method for preparing a peach blossom fermentation liquid according to claim 1, wherein in the step (4),
*Saccharomyces cerevisiae* powder is added into the fermentation substrate for the primary fermentation, and an added amount of the *Saccharomyces cerevisiae* powder is 1%-7%, preferably 3% by mass of the fermentation substrate;
the conditions of the primary fermentation are: a temperature of 30 ± 2°C, a rotation speed of 120 r/min, and a time of 4 d; and
the conditions of the sterilizing are 85°C and 20 min.

3. The method for preparing a peach blossom fermentation liquid according to claim 1, wherein in the step (5),
a composite lactic acid bacteria liquid is added into the primary fermentation liquid for the secondary fermentation, an added amount of the composite lactic acid bacteria liquid is 2%-8%, preferably 4% by volume of the fermentation substrate; in the composite lactic acid bacteria liquid, the viable bacteria count of the *Lactobacillus plantarum* is 1.5 × 10⁸-3 × 10⁸ cfu/mL; the viable bacteria count of the *Lactobacillus bulgaricus* is 2 × 10⁶ to 4 × 10⁶ cfu/mL; the viable bacteria count of the *Lactobacillus helveticus* is 1 × 10⁶-2×10⁶ cfu/mL; the viable bacteria count of the *Leuconostoc mesenteroides subsp. mesenteroides* is 1 × 10⁸ to 2 × 10⁸ cfu/mL; and the viable bacteria count of the *Streptococcus thermophilus* is 2 × 10⁶ to 4 × 10⁶ cfu/mL;
the composite lactic acid bacteria liquid is obtained by activating composite lactic acid bacteria powder in a MRS liquid medium; wherein in the composite lactic acid bacteria powder, a mass ratio of *Lactobacillus plantarum* powder, *Lactobacillus bulgaricus* powder, *Lactobacillus helveticus* powder, *Leuconostoc mesenteroides subsp. mesenteroides* powder, and *Streptococcus thermophilus* powder is 1:1.5:1:1:1.5;
the conditions of the secondary fermentation are: a temperature of 33 ± 1°C, a speed of 120 r/min, and a time of 3 d;
the conditions of the sterilizing are 85°C and 20 min;
the concentrating is to concentrate the filtered filtrate to 1/3-1/2 of the original volume.

4. The method for preparing a peach blossom fermentation liquid according to claim 1, wherein in the step (2),
an enzymolysis temperature is 40-60°C; an enzymolysis time is 60-180 min; an added amount of the flavourzyme is 0.5%-1.5% by mass of the peach blossom liquid with dregs; the added amount of the cellulase is 0.5%-2% by mass of the peach blossom liquid with dregs; and the conditions of inactivating the enzymes are 110°C and 20 min;
preferably, an enzymolysis temperature is 55°C; an enzymolysis time is 120 min; the added amount of the aminopeptidase is 1.5% by mass of the peach blossom liquid with dregs; and the added amount of the cellulase is 0.75% by mass of the peach blossom liquid with dregs.

5. The method for preparing a peach blossom fermentation liquid according to claim 1, wherein,
the step (1) further comprises: primary decoction: soaking the dried peach blossom in water, and then decocting and filtering to obtain a filtrate and a filter residue; and secondary decoction: taking the filter residue, adding with water and decocting again, filtering, and combining the filtrates and filter residues of the two times of decoction to obtain the peach blossom liquid with dregs,
in the step of primary decoction, an amount of the water added into the dried peach blossom is 20-40 times the mass of the dried peach blossom; a soaking time is 30-40 min; and the decoction process is sequentially as follows: decocting at 1,800-2,100 W for 20-40 min, decocting at 1,200-1,500 W for 20-40 min, and decocting at 600-800 W for 20-40 min;
in the step of secondary decoction, an amount of the water added into the filter residue is 10-20 times the mass of the dry peach blossom; and the decoction process is sequentially as follows: boiling at 1,800-2,100 W and then switching to decoct at 600-800 W for 40-60 min;
preferably, in the step of primary decoction, an amount of water added into the dried peach blossoms is 20 times the mass of the dried peach blossom; a soaking time is 30 min; and the decoction process is sequentially as follows: decocting at 2,100 W for 20 min, decocting at 1,400 W for 20 min, and decocting at 800 W for 20 min;
in the step of secondary decoction, an amount of the water added into the filter residue is 10 times the mass of the dry peach blossom; and the decoction process is sequentially as follows: boiling at 2,100 W and then switching to decoct at 800 W for 40 min.

6. The method for preparing a peach blossom fermentation liquid according to claim 1, wherein, in the step (3),
the carbon source is white granulated sugar, and an added amount of the white granulated sugar is 3%-20% by mass of the enzymolysis dreg-liquid mixture; and the conditions of sterilizing are 80-100°C and 5-10 min;
preferably, an added amount of the white granulated sugar is 5% by mass of the enzymolysis dreg-liquid mixture; and the conditions of sterilizing are 100°C and 5 min.

7. A peach blossom fermentation liquid obtained by the preparation method according to any one of claims 1-6.

8. A peach blossom fermentation liquid obtained by the method according to any one of claims 1-6 for use in improving constipation.

9. The peach blossom fermentation liquid for the use according to claim 8, wherein the constipation is temporary conventional constipation or slow transit constipation.

10. The peach blossom fermentation liquid for the use according to claim 9, wherein the slow transit constipation is constipation caused by an opioid drug; and the opioid drug comprises loperamide.

## Patentansprüche

1. Verfahren zur Aufbereitung eines Pfirsichblüten-Fermentationsfluids, umfassend folgende Schritte:
(1) Einweichen getrockneter Pfirsichblüten in Wasser und anschließend Abkochen unter Erhalt eines Pfirsichblüten-Fluids mit Rückständen;
(2) Zugeben von Aromatase und Cellulase zu dem Pfirsichblüten-Fluid mit Rückständen zur enzymatischen Hydrolyse sowie Inaktivieren der Enzyme unter Erhalt eines enzymatisch hydrolysierten Rückstand-Fluid-Gemisches;
(3) Zugeben einer Kohlenstoffquelle zu dem enzymatisch hydrolysierten Rückstand-Fluid-Gemisch und Sterilisieren unter Erhalt eines Fermentationssubstrats;
(4) Beimpfen des Fermentationssubstrats mit *Saccharomyces cerevisiae* zu einer primären Fermentation und Sterilisieren nach Abschluss der Fermentation unter Erhalt eines primären Fermentationsfluids; und
(5) Beimpfen des primären Fermentationsfluids mit komplexen Milchsäurebakterien zu einer sekundären Fermentation, Sterilisieren nach Abschluss der Fermentation, Filtrieren und Konzentrieren unter Erh*alt* de*s* Pfirsichb*l*ü*t*e*n*-Fermen*tatio*n*s*f*l*uid*s,* wo*b*e*i* die k*o*mplexen Milchsäur*e*bakterien *Lactobacillus plantarum, Lactobacillus bulgaricus, Lactobacillus helveticus, Leuconostoc mesenteroides subsp. mesenteroides* und *Streptococcus thermophilus* umfassen.

2. Verfahren zur Aufbereitung eines Pfirsichblüten-Fermentationsfluids nach Anspruch 1, wobei in Schritt (4) Pulver von *Saccharomyces cerevisiae* zur primären Fermentation zu dem Fermentationssubstrat zugegeben wird und die Zugabemenge des Pulvers von *Saccharomyces cerevisiae* 1% bis 7%, vorzugsweise 3%, bezogen auf eine Masse des Fermentationssubstrats, beträgt,
wobei die Bedingungen der primären Fermentation wie folgt lauten: Temperatur 30 ± 2 °C, Drehzahl 120 r/min, Dauer 4 d, und
wobei die Sterilisierungsbedingungen 85 °C und 20 min betragen.

3. Verfahren zur Aufbereitung eines Pfirsichblüten-Fermentationsfluids nach Anspruch 1, wobei in Schritt (5) ein komplexes Milchsäurebakterien-Fluid zur sekundären Fermentation zum primären Fermentationsfluid zugegeben wird, wobei die Zugabemenge des komplexen Milchsäurebakterien-Fluids 2 % bis 8 %, vorzugsweise 4 %, bezogen auf ein Volumen des Fermentationssubstrats, beträgt, wobei in dem k*o*mplexen Milchsäurebakterien-Fluid die lebensfähige Bakterienzahl von *Lactobacillus plantarum* 1,5 × 10⁸ bis 3 × 10⁸ KbE/mL, die lebensfähige Bakterienzahl von *Lactobacillus bulgaricus* 2 × 10⁶ bis 4 × 10⁶ KbE/mL, die lebensfähige Bakterienzahl von *Lactobacillus helveticus* 1 × 10⁶ bis 2 × 10⁶ KbE/mL, die lebensfähige Bakterienzahl von *Leuconostoc mesenteroides subsp. mesenteroides* 1 × 10⁸ bis 2 × 10⁸ KbE/mL und die lebensfähige Bakterienzahl von *Streptococcus thermophilus* 2 × 10⁶ bis 4 × 10⁶ KbE/mL beträgt,
wobei das komplexe Milchsäurebakterien-Fluid durch Aktivieren von komplexem Milchsäurebakterien-Pulver in einem flüssigen MRS-Medium erhalten wird, wobei in dem k*o*mplexen Milchsäure*ba*k*t*er*i*en-P*ul*ve*r* ein M*a*ssenverhältnis von Pulver von *Lactobacillus plantarum*, Pulver von *Lactobacillus bulgaricus*, Pulver von *Lactobacillus helveticus*, Pulver von *Leuconostoc mesenteroides subsp. mesenteroides* und Pulver von *Streptococcus thermophilus* 1:1,5:1:1:1,5 beträgt,
wobei die Bedingungen der sekundären Fermentation wie folgt lauten: Temperatur 33 ± 1 °C, Drehzahl 120 r/min, Dauer 3 d,
wobei die Sterilisierungsbedingungen 85 °C und 20 min betragen,
wobei das Konzentrieren darin besteht, das filtrierte Filtrat auf 1/3 bis 1/2 des ursprünglichen Volumens einzukonzentrieren.

4. Verfahren zur Aufbereitung eines Pfirsichblüten-Fermentationsfluids nach Anspruch 1, wobei in Schritt (2) eine Temperatur der enzymatischen Hydrolyse 40 bis 60 °C beträgt, wobei die Dauer der enzymatischen Hydrolyse 60 bis 180 min beträgt, wobei die Zugabemenge der Aromatase 0,5 % bis 1,5 %, bezogen auf die Masse des Pfirsichblüten-Fluids mit Rückständen, beträgt, wobei die Zugabemenge der Cellulase 0,5 % bis 2 %, bezogen auf die Masse des Pfirsichblüten-Fluids mit Rückständen, beträgt, und wobei die Inaktivierungsbedingungen der Enzyme 110 °C und 20 min betragen,
wobei die Temperatur der enzymatischen Hydrolyse vorzugsweise 55 °C beträgt, wobei die Dauer der enzymatischen Hydrolyse 120 min beträgt, wobei die Zugabemenge der Aminopeptidase 1,5 %, bezogen auf die Masse des Pfirsichblüten-Fluids mit Rückständen, beträgt, und wobei die Zugabemenge der Cellulase 0,75 %, bezogen auf die Masse des Pfirsichblüten-Fluids mit Rückständen, beträgt.

5. Verfahren zur Aufbereitung eines Pfirsichblüten-Fermentationsfluids nach Anspruch 1, wobei der Schritt (1) ferner Folgendes umfasst: ein primäres Abkochen: Einweichen der getrockneten Pfirsichblüte in Wasser, anschließend Abkochen und Filtrieren unter Erhalt eines Filtrats und eines Filterrückstands; sowie ein sekundäres Abkochen: Nehmen des Filterrückstands, Zugeben des Wassers und erneut Abkochen, Filtrieren sowie Vereinigen der Filtrate und der Filterrückstände aus den beiden Abkochvorgängen unter Erhalt des Pfirsichblüten-Fluids mit Rückständen,
wobei im Schritt des primären Abkochens die zu der getrockneten Pfirsichblüte zugegebene Wassermenge das 20- bis 40-fache der Masse der getrockneten Pfirsichblüte beträgt, wobei eine Einweichzeit 30 bis 40 min beträgt, und wobei der Abkochvorgang nacheinander wie folgt abläuft: Abkochen bei 1.800 bis 2.100 W für 20 bis 40 min, Abkochen bei 1.200 bis 1.500 W für 20 bis 40 min und Abkochen bei 600 bis 800 W für 20 bis 40 min,
wobei im Schritt des sekundären Abkochens die zu dem Filterrückstand zugegebene Wassermenge das 10- bis 20-fache der Masse der getrockneten Pfirsichblüte beträgt, wobei der Abkochvorgang nacheinander wie folgt abläuft: Aufkochen bei 1.800 bis 2.100 W und anschließend Umschalten zum Abkochen bei 600 bis 800 W für 40 bis 60 min,
wobei im Schritt des primären Abkochens die zu der getrockneten Pfirsichblüte zugegebene Wassermenge vorzugsweise das 20-fache der Masse der getrockneten Pfirsichblüte beträgt, wobei eine Einweichzeit 30 min beträgt, und wobei der Abkochvorgang nacheinander wie folgt abläuft: Abkochen bei 2.100 W für 20 min, Abkochen bei 1.400 W für 20 min und Abkochen bei 800 W für 20 min,
wobei im Schritt des sekundären Abkochens die zu dem Filterrückstand zugegebene Wassermenge das 10-fache der Masse der getrockneten Pfirsichblüte beträgt, wobei der Abkochvorgang nacheinander wie folgt abläuft: Aufkochen bei 2.100 W und anschließend Umschalten zum Abkochen bei 800 W für 40 min.

6. Verfahren zur Aufbereitung eines Pfirsichblüten-Fermentationsfluids nach Anspruch 1, wobei in Schritt (3) die Kohlenstoffquelle weißer Kristallzucker ist und die Zugabemenge des weißen Kristallzuckers 3 % bis 20 %, bezogen auf die Masse des enzymatisch hydrolysierten Rückstand-Fluid-Gemisches, beträgt, wobei die Sterilisierungsbedingungen 80 bis 100 °C und 5 bis 10 min betragen,
wobei die Zugabemenge des weißen Kristallzuckers vorzugsweise 5 %, bezogen auf die Masse des enzymatisch hydrolysierten Rückstand-Fluid-Gemisches, beträgt, wobei die Sterilisierungsbedingungen 100 °C und 5 min betragen.

7. Pfirsichblüten-Fermentationsfluid, das nach dem Aufbereitungsverfahren nach einem der Ansprüche 1 bis 6 erhalten wird.

8. Pfirsichblüten-Fermentationsfluid, das nach dem Verfahren nach einem der Ansprüche 1 bis 6 erhalten wird und zur Verwendung bei der Verbesserung von Verstopfung dient.

9. Pfirsichblüten-Fermentationsfluid zur Verwendung nach Anspruch 8, wobei die Verstopfung eine vorübergehende konventionelle Verstopfung oder eine langsam transitierende Verstopfung ist.

10. Pfirsichblüten-Fermentationsfluid zur Verwendung nach Anspruch 9, wobei die langsam transitierende Verstopfung eine durch Opioid-Arzneimittel verursachte Verstopfung ist, und wobei das Opioid-Arzneimittel Loperamid umfasst.

## Revendications

1. Procédé de préparation d'un liquide de fermentation à base de fleurs de pêcher, comprenant les étapes suivantes consistant à :
(1) faire tremper des fleurs de pêcher séchées dans de l'eau, puis les faire décocter, afin d'obtenir un liquide de fleurs de pêcher contenant des lies ;
(2) ajouter de la Flavourzyme et une cellulase au liquide de fleurs de pêcher contenant des lies pour l'enzymolyse, et inactiver des enzymes, afin d'obtenir un mélange de lie et de liquide issu de l'enzymolyse ;
(3) ajouter une source de carbone au mélange de lie et de liquide issu de l'enzymolyse, et stériliser, afin d'obtenir un substrat de fermentation ;
(4) inoculer le substrat de fermentation avec *Saccharomyces cerevisiae* en vue d'une fermentation primaire, et stériliser une fois la fermentation terminée, afin d'obtenir un liquide de fermentation primaire ; et
(5) inoculer le liquide de fermentation primaire avec des bactéries lactiques composites en vue d'une fermentation secondaire, stériliser une fois la fermentation terminée, filtrer et concentrer, afin d'obtenir le liquide de fermentation à base de fleurs de pêcher, dans lequel les bactéries lactiqu*e*s composites comprennent *Lactobacillusplantarum, Lactobacillus bulgaricus, Lactobacillus helveticus, Leuconostoc mesenteroides subsp. mesenteroides,* et *Streptococcus thermophilus.*

2. Procédé de préparation d'un liquide de fermentation à base de fleurs de pêcher selon la revendication 1, dans lequel, à l'étape (4),
de la poudre de *Saccharomyces cerevisiae* est ajoutée au substrat de fermentation pour la fermentation primaire, et une quantité ajoutée de la poudre de *Saccharomyces cerevisiae* est comprise entre 1 % et 7 %, de préférence de 3 %, en masse du substrat de fermentation ;
les conditions de la fermentation primaire sont les suivantes : une température de 30 ± 2 °C, une vitesse de rotation de 120 tr/min, et une durée de 4 jours ; et
les conditions de stérilisation sont les suivantes : à 85 °C et pendant 20 minutes.

3. Procédé de préparation d'un liquide de fermentation à base de fleurs de pêcher selon la revendication 1, dans lequel, à l'étape (5),
un liquide de bactéries lactiques composites est ajouté au liquide de fermentation primaire pour la fermentation secondaire, une quantité ajoutée du liquide de bactéries lactiques composites est comprise entre 2 % et 8 %, de préférence de 4 %, en volume du substrat de fermentation ; dans le liquide de bactéries lactiques composites, la teneur en bactéries viables de *Lactobacillus plantarum* est comprise entre 1,5 × 10⁸ et 3 × 10⁸ cfu/mL ; la teneur en bactéries viables de *Lactobacillus bulgaricus* est de 2 × 10⁶ à 4 × 10⁶ cfu/mL ; la teneur en bactéries viables de *Lactobacillus helveticus est* comprise entre 1 × 10⁶ et 2×10⁶ cfu/mL ; la teneur en bactéries viables de *Leuconostoc mesenteroides subsp. mesenteroides* est de 1 × 10⁸ à 2 × 10⁸ cfu/mL ; et la teneur en bactéries viables de *Streptococcus thermophilus* est de 2 × 10⁶ à 4 × 10⁶ cfu/mL ;
le liquide de bactéries lactiques composites est obtenu par activation de poudre de bactéries lactiques composites dans un milieu liquide MRS ; dans lequel, au sein de la poudre de bactéries lactiques composit*e*s, un rapport massique entre de la poudre *Lactobacillus plantarum*, de la poudre *Lactobacillus bulgaricus*, de la poudre *Lactobacillus helveticus,* de la poudre *Leuconostoc mesenteroides subsp. Mesenteroides* et de la poudre *Streptococcus thermophilus* est de 1:1,5:1:1:1,5 ;
les conditions de la fermentation secondaire sont les suivantes : une température de 33 ± 1 °C, une vitesse de 120 tr/min, et une durée de 3 jours ;
les conditions de stérilisation sont les suivantes : à 85 °C et pendant 20 minutes ;
la concentration consiste à concentrer le filtrat filtré jusqu'à 1/3 à 1/2 du volume initial.

4. Procédé de préparation d'un liquide de fermentation à base de fleurs de pêcher selon la revendication 1, dans lequel, à l'étape (2),
une température d'enzymolyse est comprise entre 40 et 60 °C ; une durée d'enzymolyse est comprise entre 60 et 180 min ; une quantité ajoutée de la Flavourzyme est comprise entre 0,5 % et 1,5 % en masse du liquide de fleurs de pêcher contenant des lies ; la quantité ajoutée de la cellulase est comprise entre 0,5 % et 2 % en masse du liquide de fleurs de pêcher contenant des lies ; et les conditions d'inactivation des enzymes sont les suivantes : à 110 °C et pendant 20 minutes ;
de préférence, une température d'enzymolyse est de 55 °C ; une durée d'enzymolyse est de 120 minutes ; la quantité ajoutée de l'aminopeptidase est de 1,5 % en masse du liquide de fleurs de pêcher contenant des lies ; et la quantité ajoutée de la cellulase est de 0,75 % en masse du liquide de fleurs de pêcher contenant des lies.

5. Procédé de préparation d'un liquide de fermentation à base de fleurs de pêcher selon la revendication 1, dans lequel,
l'étape (1) comprend en outre : une décoction primaire consistant à : faire tremper les fleurs de pêcher séchées dans de l'eau, puis les faire décocter et les filtrer afin d'obtenir un filtrat et un résidu de filtration ; et une décoction secondaire consistant à : prendre le résidu de filtration, y ajouter de l'eau et procéder à une nouvelle décoction, filtrer, puis combiner les filtrats et les résidus de filtration des deux décoctions afin d'obtenir le liquide de fleurs de pêcher contenant des lies,
lors de l'étape de décoction primaire, une quantité de l'eau ajoutée aux fleurs de pêcher séchées est comprise entre 20 et 40 fois la masse des fleurs de pêcher séchées ; une durée de trempage est comprise entre 30 et 40 minutes ; et le processus de décoction se déroule successivement comme suit : décoction à 1 800 à 2 100 W pendant 20 à 40 minutes, décoction à 1 200 à 1 500 W pendant 20 à 40 minutes, puis décoction à 600 à 800 W pendant 20 à 40 minutes ;
lors de l'étape de décoction secondaire, une quantité de l'eau ajoutée au résidu de filtration est comprise entre 10 et 20 fois la masse des fleurs de pêcher séchées ; et le processus de décoction se déroule successivement comme suit : ébullition à 1 800 à 2 100 W, puis passage à une décoction à 600 à 800 W pendant 40 à 60 minutes ;
de préférence, lors de l'étape de décoction primaire, une quantité d'eau ajoutée aux fleurs de pêcher séchées est de 20 fois la masse des fleurs de pêcher séchées ; une durée de trempage est de 30 minutes ; et le processus de décoction se déroule successivement comme suit : décoction à 2 100 W pendant 20 minutes, décoction à 1 400 W pendant 20 minutes, puis décoction à 800 W pendant 20 minutes ;
lors de l'étape de décoction secondaire, une quantité de l'eau ajoutée au résidu de filtration est comprise entre 10 fois la masse des fleurs de pêcher séchées ; et le processus de décoction se déroule successivement comme suit : ébullition à 2 100 W, puis passage à une décoction à 800 W pendant 40 minutes.

6. Procédé de préparation d'un liquide de fermentation à base de fleurs de pêcher selon la revendication 1, dans lequel, à l'étape (3),
la source de carbone est du sucre granulé blanc, et une quantité ajoutée du sucre granulé blanc est comprise entre 3 % et 20 % en masse du mélange de lie et de liquide issu de l'enzymolyse ; et les conditions de stérilisation sont les suivantes : à 80 à 100 °C et pendant 5 à 10 minutes ;
de préférence, une quantité ajoutée du sucre granulé blanc est de 5 % en masse du mélange de lie et de liquide issu de l'enzymolyse ; et les conditions de stérilisation sont les suivantes : à 100 °C et pendant 5 minutes.

7. Liquide de fermentation à base de fleurs de pêcher obtenu par le procédé de préparation selon l'une quelconque des revendications 1 à 6.

8. Liquide de fermentation à base de fleurs de pêcher obtenu par le procédé selon l'une quelconque des revendications 1 à 6, destiné à être utilisé pour soulager la constipation.

9. Liquide de fermentation à base de fleurs de pêcher destiné à l'utilisation selon la revendication 8, dans lequel la constipation est une constipation conventionnelle temporaire ou une constipation à transit ralenti.

10. Liquide de fermentation à base de fleurs de pêcher destiné à l'utilisation selon la revendication 9, dans lequel la constipation à transit ralenti est une constipation provoquée par un médicament opioïde ; et le médicament opioïde comprend du lopéramide.
